# EUROPEAN PATENT SPECIFICATION

(11) **EP 4 223 860 B1**
(45) Date of publication and mention of the grant of the patent: **02.04.2025**
(21) Application number: 20956146.3
(22) Date of filing: 30.09.2020
(51) Int. Cl.: C11B 1/10, C11B 3/02, C11B 13/02, C11C 1/02, C11C 1/08, C11C 3/04, C11B 3/12, C11C 1/10, C11C 3/00

(54) **METHOD FOR OBTAINING CHOLESTEROL PRESENT IN FISH OIL**
VERFAHREN ZUR GEWINNUNG VON CHOLESTERIN AUS FISCHÖL
PROCÉDÉ D'OBTENTION DE CHOLESTÉROL PRÉSENT DANS L'HUILE DE POISSON

(43) Date of publication of application: 09.08.2023
(73) Proprietor: Naturmega S.A., Barranquilla 080001 (CO)
(72) Inventor: MARTÍNEZ GUTIÉRREZ, Wilson, Barranquilla 080001 (CO); PUCHE SIMANCA, Alfredo de Jesús, Barranquilla 080001 (CO); YEPES BUSTILLO, Tatiana Lucía, Barranquilla 080001 (CO); GARCIA PADILLA, Álvaro José, Barranquilla 080001 (CO); BERRIO ROA, Lorena, Barraquilla 080001 (CO)
(74) Representative: Schüssler, Andrea
(86) International application number: PCT/IB2020/059184
(87) International publication number: WO 2022/069923

(56) References cited:
- WO-A1-2016/096989
- WO-A1-2019/159020
- WO-A1-97/24420
- WO-A2-2010/010364
- ES-A1- 2 395 320
- JP-A- S5 315 308
- US-B1- 10 196 583

## Description

### FIELD OF THE INVENTION

The present invention relates to a process for separating the cholesterol present in fish oil. Particularly, it comprises a process for separating the cholesterol present in fish oil by methods of transesterification of the refined oil, molecular distillation, saponification of a heavy fraction, and extraction and purification of cholesterol through crystallization. Based on the cholesterol separation process of the invention, a concentration of contaminants which are persistent and bioaccumulate is not generated. Therefore, the present invention provides a process for obtaining pharmaceutical grade cholesterol from fish oil having at least 90% cholesterol and simultaneously producing a high quality residual or processed fish oil suitable for human or animal consumption or for processing of EPA and DHA concentrates among others at the pharmaceutical level.

Cholesterol is a sterol (or modified steroid), lipid molecule and is biosynthesized by animal cells as it is an essential structural component of animal cell membranes, which is required to maintain both membrane structural integrity and fluidity. This substance can be found in various sources, such as egg yolk, spinal cord, bones, brain tissue of cows, goat, pig, poultry, fish organs and shrimp exoskeleton and it is even possible to find it in vegetable oils in low concentrations. However, the most important source of cholesterol is lanolin, obtained from sheep's wool. These sources generally contain cholesterol in its free form, as well as in the esterified form (cholesterol molecule bound to a fatty acid).

Cholesterol is an important pharmaceutical intermediate required for the biosynthesis of steroid hormones, bile acids, and vitamin D₃. There is currently an increasing demand for pharmaceutical grade cholesterol having a cholesterol content of 90% or more for the production of vitamins D₃, vitamin D₃ derivatives, hormones and emulsions in cosmetics. Among other uses is the synthesis of anti-inflammatory steroid drugs, production of creams for skin lubrication and cosmetics. Other cholesterols with a much lower purity are used for feeding crustaceans and aquaculture.

Cholesterol can be found in egg yolks, organ meats, shrimp, calamari, beef, pork, poultry, fish, wool grease, full-fat dairy products, butter, hard margarines, lard, coconut oil , butter (clarified butter), vegetable shortening and palm oil. These sources generally contain cholesterol in its free form, as well as in the esterified form. The most important source of cholesterol is lanolin, which is obtained from sheep's wool. However, since the fatty acid composition in wool fat is complex in nature due to the presence of free acids and alcohols that can form variable esters, cholesterol extraction is cumbersome and expensive.

Crude fish oils mainly contain triglycerides that make up about 90% of the composition. The other components of the composition comprise partial glycerides (ie, mono- or diglycerides), free fatty acids, phospholipids, and a group of chemicals as the non-saponifiable fraction that includes cholesterol-sterol, glyceryl ethers, fatty alcohols, vitamins, and oxidized pigments. The content of the unsaponifiable fraction varies with seasonal and feeding conditions and varies in the range of 2-8%.

Fish oil residues from the fish refining industry contain useful products such as cholesterol, proteins, and enzymes, among other fatty acids. Due to the importance of cholesterol as a precursor in the manufacture of vitamin D₃, fish oil and fish oil residues (after removal of polyunsaturated fatty acids) that form the waste stream in the fish oil industry are now considered as an alternative source of cholesterol.

Due to the importance of cholesterol as an intermediate in the production of vitamin D and its derivatives, there is always a need to provide alternative and improved isolation methods for cholesterol production.

### BACKGROUND OF THE INVENTION

Currently, cholesterol on an industrial scale is produced primarily from wool wax alcohols, ie, the unsaponifiable fraction of wool fat, which contains from about 25% to about 32% cholesterol. The most common processes for producing cholesterol involve the formation of an insoluble addition product by reacting cholesterol with a metal salt, followed by decomposition and recovery of the cholesterol. Such processes can meet the purity requirements for cholesterol applications in the pharmaceutical industry. In this sense, the state of the art has presented a variety of disadvantages. In particular, some prior art examples include United States patent US10190074 by Meza et al., which discloses a process for producing a cholesterol concentrate that includes the steps of distilling a fish oil that has no more than 2% free fatty acids in a mixture with an auxiliary fluid in a vacuum distillation column to obtain a first distillate and a first residue; and distilling the first distillate in a vacuum distillation column to obtain a second distillate and a second residue, wherein the second residue includes the cholesterol concentrate. These prior art processes of using high temperatures during molecular distillation generates degradation of the omega-3 fatty acids such as EPA (eicosapentanoic acid) and DHA (docosahexanoic acid), so that the content of trans fatty acids is increased and further promotes the polymerization of unsaturated fatty acids.

The same happens with the teachings of Great Britain patent GB489623A that uses a material that contains a compound that has a cholesteric nucleus, such as sterols, saponins and bile acids, which are subjected to a short path high vacuum distillation to separate the substance thereof, and then the compound is purified. Specified raw materials include animal oils and waxes, such as whale oil, fecal fats and oils, and china wax; vegetable oils, such as soybean oil, cottonseed oil, wheat germ oil, and bean oil; and fungal growths, such as ergot, each of which are sources of zoosterols, phytosterols, and mycosterols, respectively. In the same way, in Japanese patent JPS62145099A by Hata et al., which discloses a fish oil, preferably subjected to degumming treatment, obtained from a fish such as sardines, mackerel, saury fish, etc., it is subjected to molecular distillation using, for example, in an apparatus such as a falling film type, or centrifugal type.

Furthermore, the above prior art reveals temperatures that generate large concentrations of pollutants such as polychlorinated biphenyls (PCB's), polyaromatic hydrocarbons (PAH), heavy metals, pesticides and degradation products of the aforementioned compounds, which are persistent and bioaccumulative.

However, starting from a raw material other than fish oil, such as wool fat/lanolin, does not allow the recovery of fatty acids in the form of saponified soap during cholesterol esterification. The obtained soap is split to obtain free fatty acids and then esterified by means of an acid catalyst to obtain fatty acids in the form of ethyl ester. Starting from the resulting ethyl ester, it is possible to concentrate omega-3 by means of molecular distillation.

GB526951 describes a process for the extraction of cholesterol from animal tissues such as the brain, spinal cord, etc. by saponification and extraction with a water immiscible solvent. These known processes generate large amounts of liquid industrial waste, the management and/or disposal of which significantly increases production costs.

WO 97/24420 A1 concerns polyunsaturated fatty acid and fatty acid ester mixtures free of sterols and phosphorous compounds and a process for removing sterols and phosphorous compounds from naturally occurring lipid mixtures.

WO 2019/159020 A1 concerns a 6-step process for producing cholesterol from fish oil.
Given the problems of the prior art, the inventors have discovered that cholesterol can be extracted with high yields and good quality from fish oil by ultrasonic transesterification, molecular distillation, saponification, solvent extraction and cholesterol purification methods through crystallization.

### OBJECTS OF THE INVENTION

Therefore, a first object of the present invention is to avoid the drawbacks of the prior art. Particularly, the main object of the present invention is to create a process for separating the cholesterol present from fish oil, which process does not generate any amounts of contaminants.

According to the present invention, the main object of the invention is to provide beneficial processes wherein cholesterol purity of at least 95% is obtained, wherein such processes can be conducted on an industrial scale capable of satisfying the market demand for pharmaceutical grade cholesterol, as the raw material. It is also commonly easy to obtain and provide an alternative for use of a co-product used to feed crustaceans, which production cost is low and the solvents used are recovered and reused in the production process.

Another no less important object is the separation of cholesterol present in the heavy fraction from fish oil by methods of saponification and extraction and purification of cholesterol through crystallization.

Finally, another object of the invention also resides in the relationship with molecular distillation or short-path distillation. This is an operation technique for liquid-liquid separation done continuously under vacuum pressure. Considering a high vacuum pressure, the distance between the heating surface and the surface of the internal condenser of the evaporator is less than or equal to the mean free path of the molecules during the separation. In this process, a thin film is formed on the hot surface and the most volatile compounds evaporate, condensing when they come into contact with the internal condenser of the evaporator. The present invention meets these needs and provides other related advantages, including obtaining pharmaceutical grade cholesterol from fish oil with a purity of at least 95%.

The novel features which are considered to be the basis of the invention are set forth in particular in the appended claims and the additional advantages thereof, will be better understood from the following detailed description of the preferred embodiments.

### DETAILED DESCRIPTION OF THE INVENTION

The present invention relates to a process for separating cholesterol present in fish oil by methods of transesterification, molecular distillation, saponification, extraction and purification of cholesterol through crystallization. The present invention relates to a process as defined in independent claim 1. Preferred embodiments are defined in the dependent claims.

In one embodiment, the invention then refers to the process based on the separation of cholesterol present in fish oil by the method of transesterification, molecular distillation, saponification, solvent extraction and purification through crystallization and obtaining cholesterol in crystalline form.

In one aspect, the present invention refers to a process for producing cholesterol from fish oil, which generally comprises the steps of esterifying the refined oil with an acid value of less than 2 mg KOH/g using alcohol under the action of a basic catalyst with the aim of converting the glycerides present in the oil to the ethyl ester form or other esters.

The alcohol used for the present invention may be selected from the group consisting of methanol, propanol, butanol and ethanol, and the catalyst may be selected from the group consisting of sodium hydroxide, potassium hydroxide, sodium
ethoxide or sodium methoxide.

Next, the process of the invention comprises concentrating the cholesterol present in the ethyl ester obtained by using a series of short-path evaporators, and then obtaining in turn another stream concentrated in EPA and DHA.

Subsequently, dissolve the concentrated cholesterol in a base in a determined amount in water with the addition of an alcohol. The base used in the present invention may comprise, without being limited to, potassium hydroxide, calcium hydroxide, sodium hydroxide, or calcium chloride.

Subsequently the mixture is saponified for a period of time and temperature with constant stirring and reflux. Subsequently, a solvent is added with stirring and alcohol is added to promote correct separation. The above step is followed by a centrifugation process to separate the soap from the extracted phase.

In other aspects of the invention, it is important that a new extraction with a second solvent must be carried out with stirring and addition of alcohol to increase the separation of the unsaponifiable matter contained in the solvent. The solvent is then evaporated using a thin film evaporator to obtain dry unsaponifiable matter. Subsequently, a wash is carried out with a solution of sodium hydroxide or calcium chloride and it is then centrifuged to eliminate the washing water and then a solvent such as acetone, benzene or toluene is added and a first crystallization is carried out. A second solvent selected from the group consisting of hexane, diethyl ether, petroleum ether, acetone and benzene may also be used. Centrifugation and filtration are then carried out and a second crystallization of the previously obtained crystals is carried out with an organic solvent such as diethyl ether or petroleum ether or hexane.
Finally, a new centrifugation and filtration is carried out to obtain cholesterol crystals with a purity greater than 95%.

On the other hand, the soap obtained from the last centrifugation is broken down with an acid in the presence of water at a given temperature and reaction time with agitation. Then, it is cooled and the water is separated from the free fatty acids using a centrifuge. Acids suitable for this step include sulfuric, hydrochloric, citric, acetic or phosphoric acid.

The free fatty acids are then pumped to an ultrasonic reactor, where they are esterified with an alcohol and an acid catalyst at a given temperature and reaction time with constant stirring and reflux to obtain fatty acids in the form of the ethyl ester. Said acid catalyst can be selected without being limited from the group consisting of citric acid, acetic acid, phosphoric acid, hydrochloric acid, p-toluenesulfonic acid or sulfuric acid.

Therefore, the invention initially provides a transesterification and molecular distillation process that includes a first stage for obtaining fatty acids in the form of the ethyl esters and which comprises:
i. A first step of transesterifying refined fish oil with an acid value of less than 2 mg KOH/g by reacting with methanol, propanol, butanol or ethanol in a ratio between 1:5 and 1:2 w/w with respect to the oil refined, preferably 1:3 w/w with the addition of a basic catalyst such as sodium hydroxide, potassium hydroxide, sodium ethoxide or sodium methoxide in a concentration between 0.75 and 1.5% w/w with respect to the oil, said reaction conducted in four ultrasound reactors arranged in series for a time between 20 and 60 minutes, at a pressure between 1 and 3 bar, at a temperature between 60° C and 90° C and a constant reflux of alcohol;
ii. A second step to recover the alcohol not consumed in the reaction by flash evaporation in an evaporator that operates between 0.2 and 1 bar of pressure and a temperature between 50 and 80 °C, where the alcohol recovered in this step is reusable in the first step;
iii. A third step to separate the mixture of ethyl ester and glycerin obtained from the transesterification by means of a disc centrifuge that operates between 3500 and 6000 RPM, and the reaction reaches a conversion of glycerides to ethyl ester of at least 95%;
iv. A fourth step of concentrating the cholesterol present in the oil in the form of ethyl ester through molecular distillation by obtaining a heavy fraction, using short-path distillation units arranged in series, where the residue from one evaporator is fed to the next distillation unit and the distillate it is then returned to the previous still, where the operating conditions of the stills comprise vacuum pressures from 0.1 to 10 Pa, temperatures between 120 and 165° C and heat transfer areas between 2 and 5 m² and where in this step a stream concentrated in cholesterol in the form of ester and another ethyl ester stream concentrated in EPA and DHA fatty acids are obtained.

Regarding the fourth step, it must be understood that according to the process of the invention, two streams are obtained: (a) one concentrated in cholesterol in the form of ester and (b) another concentrated in EPA and DHA.

The invention continues with a second stage of saponification and extraction that includes:
v. A fifth step to obtain a soap using a base selected from the group consisting of calcium hydroxide, sodium hydroxide, potassium hydroxide or calcium chloride in water with a concentration between 10% and 30% w/w, and also adding an alcohol such as ethanol, methanol, glycerin or propylene glycol with a ratio between 2 to 5% w/w, preferably 3% w/w with respect to the base solution in a high-efficiency mixer, where it is mixed with the concentrated fraction of cholesterol in weight ratio between 1:0.9; 1:1.1; 1:1.2, preferably 1:1 with respect to the stream coming from molecular distillation and where the mixture enters a reactor where it is saponified in a time between 1 and 4 hours, preferably 1 to 2 hours, at a temperature between 60° C and 85° C with stirring at speeds from 500 to 1050 RPM with constant reflux;
vi. A sixth extraction step where the soap from the fifth step enters an extractor column, in which a solvent selected from the group consisting of toluene, ethylene dichloride, methylene chloride, preferably ethylene dichloride, is added at a 4:1 ratio, or 1:1, preferably 3:1 with respect to the added soap, with constant stirring at a speed between 200 RPM and 750 RPM, and at a temperature between 30-50°C;
vii. A seventh step to favor the subsequent separation of phases which step includes the addition of ethanol, methanol, propanol or butanol in a concentration of 0 to 5% by weight with respect to the mixture of the extraction solvent and the soap;
viii. An eighth step that comprises centrifuging the extraction mixture to separate the soap and the solvent at a speed range between 500 and 3500 rpm, preferably 1000 rpm;
ix. A ninth step which comprises a new extraction of the centrifuged soap from the eighth step in an extractor column, in which diethyl ether, petroleum ether, acetone, benzene or hexane are added, at a 4:1 to 1:1 ratio, with respect to the fed soap, with constant agitation and at a temperature between 30 to 55 °C and accompanied by the favoring of phase separation of the seventh step, where it also includes the centrifugation of the extraction mixture based on the eighth step.
x. A tenth step that includes performing the steps from the sixth to ninth steps between 1 to 3 more times;
xi. An eleventh step where the solvent from the dispersing phases of each one of the centrifugations contemplated in the eighth, ninth and tenth steps is evaporated and recovered by means of a battery of evaporators arranged in series, which evaporators operate under pressures from 10 to 100 Pa and at temperatures between 30 to 120 °C, where after evaporation, the unsaponifiable matter (MI) that was dissolved in the solvent is recovered;
xii. A twelfth step where the soap obtained from the last centrifugation of the tenth step is arranged for a breakdown with an acid selected from the group consisting of sulfuric acid, hydrochloric acid, acetic acid, phosphoric acid or citric acid in aqueous solution at 50 to 70% w/w in an ultrasonic reactor, where the ratio of soap to acid solution is between 3:1 to 1:1, preferably 2:1 and a temperature between 70°C and 90°C for 1 to 3 hours with constant stirring. Then, it is cooled and the water is separated from the free fatty acids formed using a centrifuge that operates in a range between 450 and 850 RPM;
xiii. A thirteenth step that contemplates the esterification of the free fatty acids with ultrasound using ethanol, methanol or propanol in relation to the free fatty acid by weight between 1:5 to 1:3, preferably 1:4 and an acid catalyst such as sulfuric acid, p-toluenesulfonic acid, citric acid, acetic acid, phosphoric acid or hydrochloric acid dissolved in alcohol at a concentration of 3 to 8% w/w for a time of one to three hours at a temperature between 65 °C and 85 °C with stirring and constant reflux. Finally, a stream of fatty acids in the form of ethyl ester is obtained.

After obtaining cholesterol, it is necessary to purity it. For this purpose, the process additionally includes a third stage that includes:
xiv. A fourteenth step where the unsaponifiable matter is washed with an aqueous solution of sodium hydroxide or calcium chloride at 5-10%. The washing is carried out in a proportion of 1:2 and 1:4 by weight with respect to the desolventized unsaponifiable matter in a temperature range between 40 and 60 °C;
xv. A fifteenth step that includes centrifugation of the mixture obtained after washing to separate the unsaponifiable matter that was treated and the washing water, wherein this step is executed in a range of speeds between 500 and 3500 RPM, preferably 2000 RPM;
xvi. A sixteenth step where an organic solvent selected from acetone, benzene or toluene is added to the unsaponifiable material from the fifteenth step at a ratio of 15:1 to 5:1, heated to 30°C and stirred until dissolved . It is then crystallized at a temperature between -10°C and 5°C, then maintained under isothermal conditions at 5°C for 8 hours, and then cooled to -10°C for 10 hours.
xvii. A seventeenth step where it is centrifuged and filtered using a basket centrifuge to obtain cholesterol crystals with a purity greater than 70%.
xviii. An eighteenth step where another organic solvent selected from diethyl ether, petroleum ether or hexane is added to 70% purity cholesterol from the seventeenth step at a ratio of 15:1 to 5:11, heated to 30°C and it is stirred until dissolution, where it then crystallizes at a temperature between -40°C and 0°C, maintaining under isothermal conditions at -5°C for 10 hours, and then cooled to -40°C for 8 hours;
xix. A nineteenth step where it is centrifuged and filtered using a basket centrifuge to obtain cholesterol crystals with a purity greater than 95%.

For the present invention, the set of evaporators makes it possible to recover and purify the solvent used to reuse it in the extraction process. The product of this stage are crystals of unsaponifiable matter.

Based on the cholesterol separation process of the invention, a concentration of contaminants which are persistent and bioaccumulate is not generated.

Similarly, the invention can be reflected from the examples described below.

### EXAMPLE 1

1. 2000 kg of fish oil with an acid value of 1.37 mg KOH/g were transesterified with 600 kg of ethanol and 15 kg of potassium hydroxide in four ultrasonic reactors arranged in series within a period of 50 minutes, at a pressure of 3 bars, at a temperature of 65 °C and a constant reflux of alcohol;
2. The ethanol that was not consumed by the reaction was recovered in an evaporator operating at a pressure of 0.5 bar and 60°C.
3. The ethyl ester and glycerin mixture was separated using a disc centrifuge with a spin speed of 4500 RPM. Once the glycerin was removed, the analysis of partial glycerides and ethyl ester confirmed that the ethyl ester concentration was 95.9%;
4. Eight short-path stills were arranged in series, where the residue from one still is fed to the next still and the distillate is returned to the previous still, at temperature ranges between 125 and 160 °C and vacuum pressures from 0.1 to 7 Pa. At the end of this stage, two streams were obtained: a stream with a high cholesterol content and another stream with a high content of EPA and DHA fatty acids.
5. 100 kg of the stream with high concentration of cholesterol was obtained, which was then saponified using 100 kg of an aqueous solution of sodium hydroxide at 15% w/w, and the addition of 2 kg of propylene glycol. The reaction time was two hours at a temperature of 75 °C, and stirring at 750 RPM and with constant reflux;
6. The obtained soap then enters an extractor column in which a liquid-liquid extraction is carried out using toluene in a ratio of 3 to 1 by weight with respect to the soap that is fed into the system. The extraction is carried out with constant stirring at 400 RPM and a temperature of 40 C.
7. 1% ethanol w/w is added with respect to the soap and a solvent mixture is obtained for subsequent separation.
8. The extraction mixture is centrifuged for the separation of soap and solvent using a disc centrifuge at 1000 RPM;
9. A new extraction of the previously extracted soap was carried out in another extracting column using acetone in a 3:1 ratio with respect to the soap being fed, with constant stirring at 350 RPM and at a temperature of 45°C. In addition, it was then centrifuged at 1000 RPM to separate the solvent from the soap.
10. Additionally, three extractions and three more centrifugations were carried out as previously described.
11. The solvent from the dispersing phases of each of the centrifugations conducted in the previous steps was evaporated and recovered by means of a battery of evaporators arranged in series, which are operated under pressures from 10 to 80 Pa and temperatures between 40 to 90 °C. , wherein after evaporation, the unsaponifiable matter (MI) that was dissolved in the solvent was obtained.
12. The soap obtained from the last centrifugation conducted in the tenth step, that is, the most depleted soap, was arranged for treatment with an acid selected from citric acid in an aqueous solution at 67% w/w in an ultrasonic reactor, where the ratio of soap with respect to the acid solution was 2:1 by weight and at a temperature of 75°C for 2 hours with constant stirring. Then, the resulting reaction mixture was cooled using water from a cooling tower and the water was separated from the free fatty acids formed using a centrifuge that operated at 650 RPM.
13. The esterification of the free fatty acids was carried out in an ultrasonic reactor using ethanol, feeding 1 part of activated ethanol for every 4 parts by weight of free fatty acids. Ethanol activation was performed by dissolving 7.5% p-toluenesulfonic acid in alcohol. The reaction was carried out for 1.5 hour at a temperature of 70 °C with stirring and a constant reflux of ethanol. Finally, a stream of fatty acids was obtained in the form of ethyl ester.
14. Additionally, a wash was carried out on the previously obtained unsaponifiable matter, using a 10% aqueous solution of calcium chloride. The washing was carried out in a ratio of 1:3 by weight with respect to the desolventized unsaponifiable matter, at a washing temperature of 45 °C.
15. The separation of the washing solution was carried out by centrifugation, which stage was carried out at 2000 RPM.
16. 12 parts of acetone were then added for each part by weight of unsaponifiable matter. It was then heated to 30°C and stirred until dissolved. Then it was crystallized between 5 °C and -10 °C, maintaining isothermal conditions at 5 °C for 8 hours, then cooling to -10 °C and further maintaining isothermal conditions at this temperature for 10 hours.
17. The resulting product was centrifuged and filtered using a basket centrifuge to obtain cholesterol crystals with a purity of 74.5%.
18. Then 12 parts of petroleum ether were added to each part of cholesterol with a purity greater than 70%, and then the mixture was heated to 30°C and stirred until dissolution, where it then crystallized at a temperature between -40°C and 0 °C, maintaining isothermal conditions at -5°C for 10 hours, then cooling to -40 °C and then further maintaining isothermal conditions at this temperature for 8 hours.
19. Finally, the separation of the crystals obtained from the solvent is carried out using a basket centrifuge in to obtain cholesterol crystals with a purity of 96.5%.

### EXAMPLE 2

1. 2000 kg of fish oil with an acid value of 1.37 mg KOH/g were transesterified with 600 kg of ethanol and 15 kg of sodium ethoxide in four ultrasonic reactors arranged in series within a period of 50 minutes, at a pressure of 3 bars, at a temperature of 65 °C and a constant reflux of alcohol.
2. The ethanol that was not consumed by the reaction was recovered in an evaporator operating at a pressure of 0.5 bar and 60°C.
3. The ethyl ester and glycerin mixture was separated using a disc centrifuge with a spin speed of 4500 RPM. Once the glycerin was removed, the analysis of partial glycerides and ethyl ester confirmed that the ethyl ester concentration was 96.9%.
4. Eight short-path stills were arranged in series, where the residue from one still is fed to the next still and the distillate is returned to the previous still, at temperature ranges between 125 and 160 °C and vacuum pressures from 0.1 to 7 Pa. At the end of this stage, two streams were obtained: a stream with a high cholesterol content and another stream with a high content of EPA and DHA fatty acids.
5. 100 kg of the stream with a high concentration of cholesterol was obtained, which was thensaponified using 100 kg of an aqueous solution of potassium hydroxide at 15% w/w. The reaction time was two hours at a temperature of 75 °C, stirring at 750 RPM and with constant reflux.
6. The obtained soap then enters an extractor column in which a liquid-liquid extraction is carried out using ethylene dichloride in a ratio of 3 to 1 by weight with respect to the soap being fed. The extraction is carried out with constant stirring at 400 RPM and a temperature of 40°C.
7. The extraction mixture is centrifuged for the separation of soap and solvent in a disc centrifuge at 1000 RPM;
8. A new extraction of the previously extracted soap was then carried out in another extractor column using petroleum ether in a 3:1 ratio with respect to the soap being fed, using constant stirring at 350 RPM, accompanied by the addition of 1% ethanol for favoring the subsequent separation of the phases. In addition, it was then centrifuged at 1000 RPM to separate the solvent from the soap.
9. Three extractions and three more centrifugations were further carried out as previously described.
10. The solvent of the dispersing phases from each of the centrifugations conducted in the previous steps was evaporated and recovered by means of a battery of evaporators arranged in series, which operated under pressures from 10 to 80 Pa and at temperatures between 40 to 90 °C., wherein after evaporation, the unsaponifiable matter (MI) that was dissolved in the solvent was obtained.
11. The soap obtained from the last centrifugation of the tenth step, that is, the most spent soap, was arranged for acidification with an acid selected from citric acid in an aqueous solution at 67% w/w in an ultrasonic reactor, where the ratio of soap to with respect to the acid solution was 2:1 by weight. The process is conducted at a temperature of 75°C for 2 hours with constant stirring. Then, it was cooled using water from a tower and the water was separated from the free fatty acids that were formed using a centrifuge that operated at 650 RPM.
12. The esterification of the free fatty acids was carried out in an ultrasonic reactor using ethanol, feeding 1 part of activated ethanol for every 4 parts by weight of free fatty acids. The ethanol activation was carried out by dissolving 7.5% sulfuric acid in the alcohol. The reaction was carried out for 1.5 hour at a temperature of 70 °C with stirring and constant reflux of ethanol. Finally, a stream of fatty acids was obtained in the form of ethyl ester.
13. Additionally, a further washing step was carried out on the previously obtained unsaponifiable matter, using a 10% aqueous sodium hydroxide solution. The washing was carried out in a ratio of 1:3 by weight with respect to the desolventized unsaponifiable matter at a washing temperature of 45 °C.
14. The separation of the washing solution was carried out by centrifugation, which centrifugation was carried out at 2000 RPM.
15. 12 parts of toluene were then added for each part by weight of unsaponifiable matter, and then it was heated to 30°C and stirred until dissolved. Then it was crystallized between 5 °C and -10 °C, under isothermal conditions at 5 °C for 8 hours, then cooled to -10 °C under isothermal conditions at this temperature for 10 hours.
16. The resulting product of step 15 was then centrifuged and filtered using a basket centrifuge to obtain cholesterol crystals with a purity of 66.4%.
17. 12 parts of petroleum ether were then added to each part of cholesterol with a purity greater than 70%, and the mixture was heated to 30°C and stirred until dissolution, wherein it then crystallized at a temperature between -40°C and 0 C , and then maintaining isothermal conditions at -5 C for 10 hours, and then cooling to -40 °C under isothermal conditions at this temperature for 8 hours.
18. Finally, the separation of the crystals obtained from the solvent is carried out using a basket centrifuge in order to obtain cholesterol crystals having a purity of 92.1%.

### EXAMPLE 3

1. 2000 kg of fish oil with an acid value of 1.37 mg KOH/g were transesterified with 600 kg of ethanol and 15 kg of sodium ethoxide in four ultrasonic reactors arranged in series within a period of 50 minutes, at a pressure of 3 bar, at a temperature of 65 °C and a constant reflux of alcohol.
2. The ethanol that was not consumed by the reaction was recovered in an evaporator that operates at a pressure of 0.5 bar and 60°C.
3. The ethyl ester and glycerin mixture was separated using a disc centrifuge using a spin speed of 4500 RPM. Once the glycerin was removed, the analysis of partial glycerides and ethyl ester confirmed that the ethyl ester concentration was 96.3%.
4. Eight short-path stills were then arranged in series, where the residue from one still is fed to the next still and the distillate is returned to the previous still. The stills are operated at temperature ranges between 125 and 160 °C and at vacuum pressures from 0.1 to 7 Pa. At the end of this stage, two streams were obtained: a stream with a high cholesterol content and another stream with a high content of EPA and DHA fatty acids.
5. 100 kg of the stream with a high concentration of cholesterol which was obtained, was saponified using 100 kg of an aqueous solution of potassium hydroxide at 15% w/w. The reaction time was two hours at a temperature of 75 °C, stirring at 750 RPM and with constant reflux.
6. The obtained soap then enters an extractor column in which a liquid-liquid extraction is carried out using toluene in a ratio of 3 to 1 by weight with respect to the soap being fed. The extraction is carried out using constant stirring at 400 RPM and a temperature of 40°C.
7. The extraction mixture is then centrifuged for the separation of soap and solvent using a disc centrifuge at 1000 RPM.
8. A new extraction of the previously extracted soap was carried out in another extracting column using acetone in a 3:1 ratio with respect to the soap being fed, with constant stirring at 350 RPM, accompanied by the addition of 1% ethanol to promote subsequent phase separation. In addition, it was then centrifuged at 1000 RPM to separate the solvent from the soap.
9. Three extractions and three more centrifugations were carried out as previously described above.
10. The solvent of the dispersing phases of each of the centrifugations conducted in the previous steps was evaporated and recovered by means of a battery of evaporators arranged in series, which evaporators operated under pressures from 10 to 80 Pa and temperatures between 40 to 90 °C. After evaporation, the unsaponifiable matter (MI) that was dissolved in the solvent was obtained.
11. The soap obtained from the last centrifugation of the tenth step, that is, the most spent soap, was arranged for acidification with an acid selected from citric acid in an aqueous solution at 67% w/w in an ultrasonic reactor, where the ratio of soap to with respect to the acid solution was 2:1 by weight and a temperature of 75°C for 2 hours with constant stirring. Then, it was cooled using water from a cooling tower and the water was separated from the free fatty acids formed using a centrifuge that operated at 650 RPM.
12. The esterification of the free fatty acids was carried out in an ultrasonic reactor using ethanol, feeding 1 part of activated ethanol for every 4 parts by weight of free fatty acids. The ethanol activation was carried out by dissolving 7.5% sulfuric acid in the alcohol. The reaction was carried out for 1.5 hour at a temperature of 70 °C with stirring and constant reflux of ethanol. Finally, a stream of fatty acids was obtained in the form of the ethyl ester.
13. Additionally, a wash was carried out on the previously obtained unsaponifiable matter, using a 10% aqueous sodium hydroxide solution. The washing was carried out in a ratio of 1:3 by weight with respect to the desolventized unsaponifiable matter, and the washing temperature was 45 °C.
14. The separation of the washing solution was carried out by centrifugation and this stage was carried out at 2000 RPM.
15. 12 parts of acetone were added for each part of unsaponifiable matter, then it was heated to 30°C and stirred until dissolved. Then it was crystallized between 5 °C and -10 °C, under isothermal conditions at 5 °C for 8 hours, then cooled to -10 °C and maintaining isothermal conditions at this temperature for 10 hours.
16. It was then centrifuged and filtered using a basket centrifuge to obtain cholesterol crystals with a purity of 63.5%.
17. 12 parts of petroleum ether were added to each part of cholesterol with a purity greater than 70%, and the mixture was heated to 30°C and stirred until dissolution, where it then crystallized at a temperature between -40°C and 0°C, and further maintaining it under isothermal conditions at -5°C for 10 hours, then cooling to -40°C and then maintaining isothermal conditions at this temperature for 8 hours.
18. Finally, the separation of the crystals obtained from the solvent is carried out using a basket centrifuge in order to obtain cholesterol crystals with a purity of 94.1%.

### EXAMPLE 4

1. 2000 kg of fish oil with an acid value of 1.37 mg KOH/g were transesterified with 600 kg of ethanol and 15 kg of potassium hydroxide in four ultrasonic reactors arranged in series within a period of 50 minutes, at a pressure of 3 bars , at a temperature of 65 °C and constant reflux of alcohol;
2. The ethanol that was not consumed by the reaction was recovered in an evaporator that operates at a pressure of 0.5 bar and 60°C.
3. The ethyl ester and glycerin mixture was separated using a disc centrifuge with a spin speed of 4500 RPM. Once the glycerin was removed, the analysis of partial glycerides and ethyl ester confirmed that the ethyl ester concentration was 94.9%.
4. Eight short-path stills were arranged in series, where the residue from one still is fed to the next still and the distillate is returned to the previous still, at temperature ranging between 125 and 160 °C and vacuum pressures from 0.1 to 7 Pa. At the end of this stage, two streams were obtained: a stream with a high cholesterol content and another stream with a high content of EPA and DHA fatty acids.
5. 100 kg of the stream with high concentration of cholesterol were obtained, which was saponified using 100 kg of an aqueous solution of potassium hydroxide at 15% w/w, and the addition of 2 kg of ethanol. The reaction time was two hours at a temperature of 75 °C, with stirring at 750 RPM and with constant reflux.
6. The soap obtained then enters an extractor column in which a liquid-liquid extraction is carried out using ethylene dichloride in a ratio of 3 to 1 by weight with respect to the soap being fed. The extraction is carried out with constant stirring at 400 RPM and a temperature of 40°C.
7. The extraction mixture is centrifuged for the separation of soap and solvent in a disc centrifuge at 1000 RPM.
8. A new extraction of the previously extracted soap was carried out in another extraction column using hexane in a 3:1 ratio with respect to the soap being fed, with constant stirring at 350 RPM and at a temperature of 45°C. In addition, it was centrifuged at 1000 RPM to separate the solvent from the soap.
9. Three extractions and three more centrifugations were carried out as previously described above.
10. The solvent from the dispersing phases of each of the centrifugations contemplated in the previous steps was evaporated and recovered by means of a battery of evaporators arranged in series, which operated under pressures from 10 to 80 Pa and at temperatures between 40 to 90 °C, where after evaporation, the unsaponifiable matter (MI) that was dissolved in the solvent was obtained.
11. The soap obtained from the last centrifugation of the tenth step, that is, the most spent soap, was arranged for acidification with an acid selected from citric acid in an aqueous solution at 67% w/w in an ultrasonic reactor, where the ratio of soap to the acid solution was 2:1 by weight and at temperature of 75°C for 2 hours with constant stirring. Then, it was cooled using water from a tower and the water was separated from the free fatty acids formed using a centrifuge that operated at 650 RPM.
12. The esterification of free fatty acids was carried out in an ultrasonic reactor using ethanol, by feeding 1 part of activated ethanol for every 4 parts by weight of free fatty acids. Ethanol activation was performed by dissolving 7.5% p-toluenesulfonic acid in alcohol. The reaction was carried out for 1.5 hour at a temperature of 70 °C with stirring and a constant reflux of ethanol. Finally, a stream of fatty acids was obtained in the form of the ethyl ester.
13. Additionally, a wash was carried out on the previously obtained unsaponifiable matter, using a 10% aqueous solution of calcium chloride. The washing was carried out in a ratio of 1:3 by weight with respect to the desolventized unsaponifiable matter, at a washing temperature of 45 °C;
14. The separation of the washing solution was carried out by centrifugation, and this stage was carried out at 2000 RPM.
15. 12 parts of hexane were added for each part of unsaponifiable matter, then heated to 30°C and stirred until dissolved. Then it was crystallized between 5 °C and -10 °C, then maintained under isothermal conditions at 5 °C for 8 hours, then cooling to -10 °C and further maintaining it under isothermal conditions at this temperature for 10 hours.
16. It was then centrifuged and filtered using a basket centrifuge to obtain cholesterol crystals with a purity of 74.5%.
17. 12 parts of hexane were then added to each part of cholesterol with a purity greater than 70%, and the mixture was heated to 30°C and stirred until dissolved, where it then crystallized at a temperature between -40°C and 0 °C, and then maintaining isothermal conditions at -5°C for 10 hours, then cooling to -40 °C and further maintaining isothermal conditions at this temperature for 8 hours.
18. Finally, the separation of the crystals obtained from the solvent is carried out using a basket centrifuge in order to obtain cholesterol crystals with a purity of 90.2%.

### EXAMPLE 5

1. 2000 kg of fish oil with an acid value of 1.37 mg KOH/g were transesterified with 600 kg of ethanol and 15 kg of sodium ethoxide in four ultrasonic reactors arranged in series within a period of 50 minutes, at a pressure of 3 bar , with a temperature of 65 °C and a constant reflux of alcohol.
2. The ethanol that was not consumed by the reaction was recovered in an evaporator that operates at a pressure of 0.5 bar and 60°C.
3. The ethyl ester and glycerin mixture was separated using a disc centrifuge with a spin speed of 4500 RPM. Once the glycerin was removed, the analysis of partial glycerides and ethyl ester confirmed that the ethyl ester concentration was 96.3%.
4. Eight short-path stills were arranged in series, where the residue from one still is fed to the next still and the distillate is returned to the previous still, at temperature ranging between 125 and 160 °C and vacuum pressures from 0.1 to 7 Pa. At the end of this stage, two streams were obtained: one stream with a high cholesterol content and another stream with a high content of EPA and DHA fatty acids.
5. 100 kg of the stream with a high concentration of cholesterol was obtained, which was then saponified using 100 kg of an aqueous solution of sodium hydroxide at 15% w/w. The reaction time was two hours at a temperature of 75 °C, with stirring at 750 RPM and with constant reflux.
6. The soap obtained then enters an extractor column in which a liquid-liquid extraction is carried out using toluene in a ratio of 2 to 1 by weight with respect to the soap being fed. The extraction is carried out with constant stirring at 400 RPM and at a temperature of 40°C.
7. The extraction mixture is centrifuged for the separation of soap and solvent in a disc centrifuge at 1000 RPM.
8. A new extraction of the previously extracted soap was carried out in another extracting column using acetone in a ratio of 2 to 1 with respect to the soap being fed, with constant stirring at 350 RPM, accompanied by the addition of 1% ethanol to promote subsequent phase separation. In addition, it was centrifuged at 1000 RPM to separate the solvent from the soap.
9. Two extractions and two more centrifugations were carried out as previously described above.
10. The solvent of the dispersing phases of each of the centrifugations contemplated in the previous steps was evaporated and recovered by means of a battery of evaporators arranged in series, which are operated under pressures from 10 to 80 Pa and at temperatures between 40 to 90 °C. , where after evaporation, the unsaponifiable matter (MI) that was dissolved in the solvent was obtained;
11. The soap obtained from the last centrifugation of the tenth step, that is, the most spent soap, was arranged for acidification with an acid selected from citric acid in an aqueous solution at 67% w/w in an ultrasonic reactor, where the ratio of soap to the acid solution was 2:1 by weight and at a temperature of 75°C for 2 hours with constant stirring. Then, it was cooled using water from a tower and the water was then separated from the free fatty acids formed using a centrifuge that operated at 650 RPM.
12. The esterification of the free fatty acids was carried out in an ultrasonic reactor using ethanol, feeding 1 part of activated ethanol for every 4 parts of free fatty acids. The ethanol activation was carried out by dissolving 7.5% sulfuric acid in the alcohol. The reaction was carried out for 1.5 hour at a temperature of 70 °C with stirring and constant reflux of ethanol. Finally, a stream of fatty acids was obtained in the form of the ethyl ester.
13. Additionally, a wash was carried out on the previously obtained unsaponifiable matter, using a 10% aqueous solution of calcium chloride. The washing was carried out in a ratio of 1:3 by weight with respect to the desolventized unsaponifiable matter, and the washing temperature was 45 °C.
14. The separation of the washing solution was carried out by centrifugation, this stage was carried out at 2000 RPM.
15. 12 parts of acetone were added for each mass part of unsaponifiable matter, it was heated to 30° C and stirred until dissolved. Then it was crystallized between 5° C and -10° C, and further maintained under isothermal conditions at 5 °C for 8 hours, then cooling to -10 °C and then further maintained under isothermal conditions at this temperature for 10 hours.
16. It was then centrifuged and filtered using a basket centrifuge to obtain cholesterol crystals with a purity of 78.6%.
17. 12 parts of acetone were then added to each part of cholesterol with a purity greater than 70%, the mixture was heated to 30°C and stirred until dissolved, where it then crystallized at a temperature between -40°C and 0 °C, while maintaining isothermal conditions at -5°C for 10 hours, then cooling to -40 °C and further maintaining isothermal conditions at this temperature for 8 hours.
18. Finally, the separation of the crystals obtained from the solvent is carried out using a basket centrifuge in order to obtain cholesterol crystals with a purity of 97.8%.

### EXAMPLE 6

1. 2000 kg of fish oil with an acid value of 1.37 mg KOH/g were transesterified with 600 kg of ethanol and 15 kg of sodium ethoxide in four ultrasonic reactors arranged in series within a period of 50 minutes, at a pressure of 3 bar, at a temperature of 65 °C and constant reflux of alcohol.
2. The ethanol that was not consumed by the reaction was recovered in an evaporator that operates at a pressure of 0.5 bar and 60°C.
3. The ethyl ester and glycerin mixture was separated using a disc centrifuge with a spin speed of 4500 RPM. Once the glycerin was removed, the analysis of partial glycerides and ethyl ester confirmed that the ethyl ester concentration was 96.3%;
4. Eight short-path stills were arranged in seriies, where the residue from one still is fed to the next still and the distillate is returned to the previous still, at temperature ranging between 125 and 160 °C and vacuum pressures from 0.1 to 7 Pa. At the end of this stage, two streams were obtained: a stream with a high cholesterol content and another stream with a high content of EPA and DHA fatty acids.
5. 100 kg of the stream with a high concentration of cholesterol were obtained, which stream was saponified using 100 kg of an aqueous solution of sodium hydroxide at 15% w/w. The reaction time was two hours at a temperature of 75 °C, stirring at 750 RPM and with constant reflux.
6. The obtained soap then enters an extractor column in which a liquid-liquid extraction is carried out using toluene in a ratio of 2 to 1 by weight with respect to the soap being fed. The extraction is carried out with constant stirring at 400 RPM and at a temperature of 40°C.
7. The extraction mixture is then centrifuged for the separation of soap and solvent in a disc centrifuge at 1000 RPM.
8. A new extraction of the previously extracted soap was carried out in another extracting column using diethyl ether in a ratio of 2 to 1 with respect to the soap being fed, with constant stirring at 350 RPM, accompanied by the addition of 1% ethanol to favor subsequent phase separation. In addition, it was centrifuged at 1000 RPM to separate the solvent from the soap.
9. Three extractions and three more centrifugations were carried out as previously described above.
10. The solvent from the dispersing phases of each of the centrifugations conducted in the previous steps was evaporated and recovered by means of a battery of evaporators arranged in series, which operated under pressures from 10 to 80 Pa and temperatures between 40 and 90 °C, wherein after evaporation, the unsaponifiable matter (MI) that was dissolved in the solvent was obtained.
11. The soap obtained from the last centrifugation of the tenth step, that is, the most spent soap, was arranged for acidification with an acid selected from citric acid in an aqueous solution at 67% w/w in an ultrasonic reactor, where the ratio of soap to the acid solution was 2:1 by weight and at a temperature of 75°C for 2 hours with constant stirring. Then, it was cooled using water from a tower and the water was then separated from the free fatty acids formed using a centrifuge that operated at 650 RPM.
12. The esterification of the free fatty acids was carried out in an ultrasonic reactor using ethanol, feeding 1 part of activated ethanol for every 4 parts of free fatty acids. Ethanol activation was performed by dissolving 7.5% p-toluenesulfonic acid in alcohol. The reaction was carried out for 1.5 hour at a temperature of 70 °C with stirring and constant reflux of ethanol. Finally, a stream of fatty acids was obtained in the form of the ethyl ester.
13. Additionally, a wash was carried out on the previously obtained unsaponifiable matter, using a 10% aqueous potassium hydroxide solution. The washing was carried out in a ratio of 1:3 by weight with respect to the desolventized unsaponifiable matter, at a washing temperature of 45° C.
14. The separation of the washing solution was carried out by centrifugation, and it was carried out at 2000 RPM.
15. 12 parts of benzene were added for each part of unsaponifiable matter, it was then heated to 30°C and stirred until dissolved. Then it was crystallized between 5 °C and - 10 °C, and maintained under isothermal conditions at 5 °C for 8 hours, then cooling to -10 °C and further maintained under isothermal conditions at this temperature for 10 hours.
16. It was then centrifuged and filtered using a basket centrifuge to obtain cholesterol crystals with a purity of 73.4%.
17. 12 parts of diethyl ether were added to each part of cholesterol with a purity greater than 70%, the mixture was then heated to 30°C and stirred until dissolved, where it then crystallized at a temperature between -40°C and 0°C, and then maintained under isothermal conditions at -5°C for 10 hours, then cooling to -40°C and further maintaining under isothermal conditions at this temperature for 8 hours.
18. Finally, the separation of the crystals obtained from the solvent is carried out using a basket centrifuge in order to obtain cholesterol crystals with a purity of 92.7%.

Therefore, an object of the present invention is to provide a process for producing cholesterol from fish oil, generally comprising the steps of transesterifying the refined oil using a basic catalyst. In the next step, there is obtained a stream concentrated in cholesterol and another stream concentrated in omega-3 fatty acids said fatty acids being EPA and DHA through molecular distillation. In the subsequent step, saponification of the cholesterol-rich stream is conducted using bases such as potassium hydroxide, sodium hydroxide, or calcium chloride. The mixture is saponified for a time and temperature with constant stirring and reflux. Several extractions with solvents such as toluene, ethylene dichloride, methylene chloride, diethyl ether, petroleum ether, acetone, benzene or hexane and centrifugations are also carried out to obtain the unsaponifiable matter. The last stream is crystallized using acetone, benzene, toluene, diethyl ether, petroleum ether or hexane to obtain cholesterol with a minimum purity of 95%. In addition, the soap obtained from saponification is converted to the ethyl ester forms by dual reactions that is acidification of the soap and then acid esterification.

## Claims

1. Cholesterol separation process from the heavy fraction of fish oil and **characterized in that** it comprises a first stage of obtaining fatty acids in the form of their esters and comprising:
(i) a first step of transesterifying refined fish oil with an acid value of less than 2 mg KOH/g from the reaction with methanol, propanol, butanol or ethanol in a ratio between 1:5 and 1:2 w/w with respect to the oil refined, preferably 1:3 w/w with the addition of a basic catalyst selected from the group consisting of sodium hydroxide, potassium hydroxide, sodium ethoxide or sodium methoxide in a concentration between 0.75 and 1.5% w/w with respect to the oil, where it is allowed react in four ultrasonic reactors arranged in series between 20 and 60 minutes, at a pressure between 1 and 3 bar, at a temperature between 60 °C and 90 °C and constant reflux of alcohol;
(ii) a second step to recover the alcohol not consumed in the reaction by flash evaporation in an evaporator that operates between 0.2 and 1 bar of pressure and temperature between 50 and 80 °C, where the alcohol recovered in this step is reusable in the first step;
(iii) a third step to separate the mixture of the ester and glycerin obtained from the transesterification by means of a disc centrifuge that operates between 3500 and 6000 RPM, wherein the reaction reaches a conversion of glycerides to esters is at least 95%;
(iv) a fourth step of concentrating the cholesterol present in the oil in the form of ester through molecular distillation obtaining a heavy fraction, using short-path distillers arranged in series, where the residue from one evaporator is fed to the next distiller and the distillate is returned to the previous still, where the operating conditions of the stills comprise vacuum pressures from 0.1 to 10 Pa, temperatures between 120 and 165°C and heat transfer areas between 2 and 5 m² and wherein in this step a stream concentrated in cholesterol content in the form of ester and another stream of esters concentrated in EPA and DHA fatty acids is obtained.

2. Cholesterol separation process from the heavy fraction of fish oil according to claim 1, wherein it comprises a second stage of obtaining saponification and extraction products that includes:
(v) a fifth step to obtain a soap by using a base selected from the group consisting of calcium hydroxide, sodium hydroxide, potassium hydroxide or calcium chloride in water with a concentration between 10% and 30% w/w, with the addition of ethanol, methanol, glycerin or propylene glycol with a ratio between 2 to 5% w/w, preferably 3% w/w with respect to the base solution in a high-efficiency mixer, where it is mixed with the concentrated fraction of cholesterol in weight ratio between 1:0.9; or 1:1.1; or 1:1.2, preferably 1:1 with respect to the stream coming from molecular distillation and where the mixture enters a reactor where it is saponified for a time between 1 and 4 hours, preferably 1 to 2 hours, at a temperature between 60 °C and 85°C with stirring between 500 to 1050 RPM using with constant reflux;
(vi) a sixth extraction step where the soap from the fifth step enters an extracting column, in which a solvent selected from the group consisting of toluene, ethylene dichloride, methylene chloride, preferably ethylene dichloride is added, with a ratio of 4:1 to 1:1, preferably 3:1 with respect to the soap being fed, with constant stirring between 200 RPM and 750 RPM, and at a temperature between 30-50°C;
(vii) a seventh step of favoring a subsequent separation of phases that includes the addition of ethanol, methanol, propanol or butanol in a concentration of 0 to 5% by weight with respect to the mixture of the extraction solvent and the soap;
(viii) an eighth step that includes centrifugation of the extraction mixture for the separation of soap and solvent at a speed range between 500 and 3500 rpm;
(ix) a ninth step which comprises a new extraction of the centrifuged soap from the eighth step in an extractor column, in which diethyl ether, petroleum ether, acetone, benzene or hexane are added, at a ratio of 4:1 to 1:1, with respect to the soap being fed, with constant agitation and at a temperature between 30 to 55 °C and accompanied by favoring the phase separation of the seventh step, wherein it also includes the centrifugation of the extraction mixture based on the eighth step;
(x) a tenth step that includes performing steps six through ninth 1 to 3 more times;
(xi) an eleventh step where the solvent from the dispersing phases of each of the centrifugations contemplated in the eighth, ninth and tenth steps is evaporated and recovered by means of a battery of evaporators arranged in series, which operate under pressures from 10 to 100 Pa and temperatures between 30 to 120 °C, wherein after evaporation, the unsaponifiable matter (MI) that was dissolved in the solvent is obtained;
(xii) a twelfth step where the soap obtained from the last centrifugation of the tenth step is arranged for acidification with an acid selected from the group consisting of sulfuric acid, hydrochloric acid, acetic acid, phosphoric acid or citric acid in an aqueous solution at 50 to 70% w/w in an ultrasonic reactor, where the ratio of soap to acid solution is between 3:1 to 1:1, preferably 2:1 and a temperature between 70°C and 90°C for 1 to 3 hours with constant stirring, then, it is cooled and the water is separated from the free fatty acids formed using a centrifuge that operates in a range between 450 and 850 RPM;
(xiii) a thirteenth step that contemplates the esterification of the free fatty acids with ultrasound using ethanol, methanol or propanol in relation to the free fatty acid by weight between 1:5 to 1:3, preferably 1:4 and an acid catalyst such as sulfuric acid , p-toluenesulfonic acid, citric acid, acetic acid, phosphoric acid or hydrochloric acid dissolved in alcohol at a concentration of 3 to 8% w/w for one to three hours at a temperature between 65 °C and 85 °C with stirring and constant reflux and finally, obtaining a stream of fatty acids in the form of their esters.

3. Cholesterol separation process from the heavy fraction of fish oil according to claims 1 and 2, wherein it comprises a third stage of cholesterol purification and crystallization that includes:
(xiv) a fourteenth step where the unsaponifiable matter is washed with an aqueous solution of sodium hydroxide or calcium chloride at 5-10%; wherein the washing is carried out in a proportion of 1:2 and 1:4 by weight with respect to the desolventized unsaponifiable matter at a temperature range between 40 and 60 °C;
(xv) a fifteenth step that includes centrifugation of the mixture obtained after washing to separate the unsaponifiable matter treated and the washing water, which step is executed at a range of speeds between 500 and 3500 RPM, preferably 2000 RPM;
(xvi) a sixteenth step where an organic solvent selected from acetone, benzene or toluene is added to the unsaponifiable material from the fifteenth step at a ratio of 15:1 to 5:1, heated to 30°C and stirred until dissolved and then crystallized at a temperature between -10°C and 5°C. and maintained under isothermal conditions at 5°C for 8 hours, then cooling to -10°C for 10 hours;
(xvii) a seventeenth step where subsequently the resulting product from the sixteenth step it is centrifuged and filtered using a basket centrifuge to obtain cholesterol crystals with a purity greater than 70%;
(xviii) an eighteenth step where another organic solvent selected from a diethyl ether, petroleum ether or hexane is added to the 70% cholesterol from the seventeenth step at a ratio of 15:1 to 5:1, heated to 30°C and stirred until dissolution, and then crystallized at a temperature between -40°C and 0°C, and then maintained under isothermal conditions at -5°C for 10 hours, and then cooled to -40°C for 8 hours; and
(xix) a nineteenth step where the product form the eighteenth step is centrifuged and filtered using a basket centrifuge and cholesterol crystals with a purity greater than 95% are obtained.

4. Cholesterol separation process present in the heavy fraction of fish oil according to claims 1, 2 and 3, wherein the washing solvent can be selected from a solution of sodium hydroxide or calcium chloride.

## Patentansprüche

1. Verfahren zur Cholesterinabtrennung aus der schweren Fraktion von Fischöl und **dadurch gekennzeichnet, dass** es eine erste Stufe zur Gewinnung von Fettsäuren in Form ihrer Ester umfasst und Folgendes umfasst:
(i) einen ersten Schritt der Umesterung von raffiniertem Fischöl mit einer Säurezahl von weniger als 2 mg KOH/g aus der Reaktion mit Methanol, Propanol, Butanol oder Ethanol in einem Gewichtsverhältnis zwischen 1:5 und 1:2, vorzugsweise 1:3 in Bezug auf das raffinierte Öl unter Zugabe eines basischen Katalysators, ausgewählt aus der Gruppe bestehend aus Natriumhydroxid, Kaliumhydroxid, Natriumethoxid oder Natriummethoxid in einer Konzentration zwischen 0,75 und 1,5 Gew.-% in Bezug auf das Öl, wobei man es in vier hintereinander geschalteten Ultraschallreaktoren zwischen 20 und 60 Minuten, bei einem Druck zwischen 1 und 3 bar, bei einer Temperatur zwischen 60°C und 90°C und einem konstanten Rückfluss von Alkohol reagieren lässt;
(ii) einen zweiten Schritt zur Rückgewinnung des bei der Reaktion nicht verbrauchten Alkohols durch Entspannungsverdampfung in einem Verdampfer, der bei einem Druck zwischen 0,2 und 1 bar und einer Temperatur zwischen 50 und 80°C arbeitet, wobei der in diesem Schritt gewonnene Alkohol im ersten Schritt wiederverwendet werden kann;
(iii) einen dritten Schritt zur Trennung des aus der Umesterung gewonnenen Gemischs aus Ester und Glycerin mittels einer Scheibenzentrifuge, die zwischen 3.500 und 6.000 U/min arbeitet, wobei die Reaktion eine Umwandlung von Glyceriden in Ester von mindestens 95% erreicht;
(iv) einen vierten Schritt der Konzentrierung des im Öl in Form von Ester vorhandenen Cholesterins durch Molekulardestillation zur Gewinnung einer schweren Fraktion unter Verwendung von in Reihe angeordneten Kurzweg-Destillierapparaten, wobei der Rückstand von einem Verdampfer dem nächsten Destillierapparat zugeführt und das Destillat in den vorherigen Destillierapparat zurückgeführt wird, wobei die Betriebsbedingungen der Destillierapparate Vakuumdrücke von 0,1 bis 10 Pa, Temperaturen zwischen 120 und 165°C und Wärmeübertragungsflächen zwischen 2 und 5 m² umfassen und, wobei in diesem Schritt ein an Cholesterin konzentrierter Strom und ein weiterer an EPA- und DHA-Fettsäuren konzentrierter Strom von Estern gewonnen wird.

2. Verfahren zur Cholesterinabtrennung aus der schweren Fraktion von Fischöl nach Anspruch 1, wobei es eine zweite Stufe zur Gewinnung von Verseifungs- und Extraktionsprodukten umfasst, die Folgendes umfasst:
(v) einen fünften Schritt zum Gewinnen einer Seife durch Verwendung einer Base, ausgewählt aus der Gruppe bestehend aus Calciumhydroxid, Natriumhydroxid, Kaliumhydroxid oder Calciumchlorid in Wasser mit einer Konzentration zwischen 10 Gew.-% und 30 Gew.-%, unter Zusatz von Ethanol, Methanol, Glycerin oder Propylenglykol mit einem Verhältnis zwischen 2 bis 5 Gew.-%, vorzugsweise 3 Gew.-% in Bezug auf die Basenlösung, in einem Hochleistungsmischer, wo sie mit der konzentrierten Cholesterinfraktion in einem von Gewichtsverhältnis zwischen 1:0,9 oder 1:1,1 oder 1:1,2; vorzugsweise 1:1 in Bezug auf den Strom, der aus der Molekulardestillation kommt, gemischt wird und, wo die Mischung in einen Reaktor gelangt, wo sie für eine Zeit zwischen 1 und 4 Stunden, vorzugsweise 1 bis 2 Stunden, bei einer Temperatur zwischen 60°C und 85°C unter Rühren zwischen 500 und 1.050 U/min unter konstantem Rückfluss verseift wird;
(vi) einen sechsten Extraktionsschritt, bei dem die Seife aus dem fünften Schritt in eine Extraktionssäule gelangt, in der ein Lösungsmittel, ausgewählt aus der Gruppe bestehend aus Toluol, Ethylendichlorid, Methylenchlorid, vorzugsweise Ethylendichlorid, in einem Verhältnis von 4:1 bis 1:1, vorzugsweise 3:1 in Bezug auf die zugeführte Seife, unter ständigem Rühren bei zwischen 200 U/min und 750 U/min und bei einer Temperatur zwischen 30-50°C zugegeben wird;
(vii) einen siebten Schritt zur Förderung einer anschließenden Phasentrennung, die die Zugabe von Ethanol, Methanol, Propanol oder Butanol in einer Konzentration von 0 bis 5 Gew.-% in Bezug auf die Mischung aus dem Extraktionslösungsmittel und der Seife umfasst;
(viii) einen achten Schritt, der das Zentrifugieren der Extraktionsmischung zur Abtrennung von Seife und Lösungsmittel bei einer Geschwindigkeit zwischen 500 und 3.500 U/min umfasst;
(ix) einen neunten Schritt, der eine erneute Extraktion der zentrifugierten Seife aus dem achten Schritt in einer Extraktionssäule umfasst, in der Diethylether, Petrolether, Aceton, Benzol oder Hexan in einem Verhältnis von 4:1 bis 1:1 in Bezug auf die zugeführte Seife unter ständigem Rühren und bei einer Temperatur zwischen 30 und 55°C und begleitet von einer Begünstigung der Phasentrennung des siebten Schritts zugegeben werden, wobei er auch die Zentrifugation des Extraktionsgemisches basierend auf dem achten Schritt umfasst;
(x) einen zehnten Schritt, der das Durchführen der Schritte sechs bis neun noch ein bis drei Mal umfasst;
(xi) einen elften Schritt, bei dem das Lösungsmittel aus den Dispergierphasen jeder der im achten, neunten und zehnten Schritt genannten Zentrifugationen verdampft und mithilfe einer Reihe von hintereinander angeordneten Verdampfern, die unter Drücken von 10 bis 100 Pa und Temperaturen zwischen 30 und 120°C arbeiten, wiedergewonnen wird, wobei nach der Verdampfung das unverseifbare Material (MI), das im Lösungsmittel gelöst wurde, gewonnen wird;
(xii) einen zwölften Schritt, bei dem die aus der letzten Zentrifugation des zehnten Schritts gewonnene Seife zur Ansäuerung mit einer Säure, ausgewählt aus der Gruppe bestehend aus Schwefelsäure, Salzsäure, Essigsäure, Phosphorsäure oder Zitronensäure, in einer wässrigen Lösung bei 50 bis 70 Gew.-% in einem Ultraschallreaktor, wobei das Verhältnis von Seife zu Säurelösung zwischen 3:1 und 1:1, vorzugsweise 2:1 beträgt, und bei einer Temperatur zwischen 70°C und 90°C für 1 bis 3 Stunden unter ständigem Rühren angeordnet wird, dann wird sie abgekühlt und das Wasser wird von den gebildeten freien Fettsäuren unter Verwendung einer Zentrifuge, die im Bereich zwischen 450 und 850 U/min arbeitet, abgetrennt;
(xiii) einen dreizehnten Schritt, der die Veresterung der freien Fettsäuren mit Ultraschall unter Verwendung von Ethanol, Methanol oder Propanol im Gewichtsverhältnis zwischen 1:5 bis 1:3, vorzugsweise 1:4, in Bezug auf die freien Fettsäuren und mit einem Säurekatalysator, wie beispielsweise Schwefelsäure, p-Toluolsulfonsäure, Zitronensäure, Essigsäure, Phosphorsäure oder Salzsäure, gelöst in Alkohol in einer Konzentration von 3 bis 8 Gew.-% für ein bis drei Stunden bei einer Temperatur zwischen 65°C und 85°C unter Rühren und ständigem Rückfluss und schließlich das Erhalten eines Stroms von Fettsäuren in Form ihrer Ester vorsieht.

3. Verfahren zur Abtrennung von Cholesterin aus der schweren Fraktion von Fischöl gemäß den Ansprüchen 1 und 2, wobei es eine dritte Stufe der Cholesterinaufreinigung und -kristallisation umfasst, die Folgendes umfasst:
(xiv) einen vierzehnten Schritt, bei dem das unverseifbare Material mit einer wässrigen Lösung von Natriumhydroxid oder Calciumchlorid bei 5-10% gewaschen wird; wobei das Waschen in einem Gewichtsverhältnis von 1:2 und 1:4 in Bezug auf das von Lösungsmittel befreite, unverseifbare Material in einem Temperaturbereich zwischen 40 und 60°C durchgeführt wird;
(xv) einen fünfzehnten Schritt, der das Zentrifugieren der nach dem Waschen gewonnenen Mischung umfasst, um das behandelte unverseifbare Material und das Waschwasser abzutrennen, wobei dieser Schritt bei einem Geschwindigkeitsbereich zwischen 500 und 3.500 U/min, vorzugsweise 2.000 U/min durchgeführt wird;
(xvi) einen sechzehnten Schritt, bei dem ein organisches Lösungsmittel, ausgewählt aus Aceton, Benzol oder Toluol dem unverseifbaren Material aus dem fünfzehnten Schritt in einem Verhältnis von 15:1 bis 5:1 zugesetzt, auf 30°C erhitzt und gerührt wird, bis es aufgelöst ist, und dann bei einer Temperatur zwischen -10°C und 5°C kristallisiert und 8 Stunden lang unter isothermen Bedingungen bei 5°C gehalten, dann 10 Stunden lang auf -10°C abgekühlt wird;
(xvii) einen siebzehnten Schritt, bei dem anschließend das resultierende Produkt aus dem sechzehnten Schritt zentrifugiert und unter Verwendung einer Korbzentrifuge filtriert wird, um Cholesterinkristalle mit einer Reinheit von mehr als 70% zu erhalten;
(xviii) einen achtzehnten Schritt, bei dem ein weiteres organisches Lösungsmittel, ausgewählt aus Diethylether, Petrolether oder Hexan, dem 70%-igen Cholesterin aus dem siebzehnten Schritt in einem Verhältnis von 15:1 bis 5:1 zugesetzt, auf 30°C erhitzt und bis zur Auflösung gerührt wird, und dann bei einer Temperatur zwischen -40°C und 0°C kristallisiert, und dann 10 Stunden lang unter isothermen Bedingungen bei -5°C gehalten, und dann 8 Stunden lang auf -40°C abgekühlt wird; und
(xix) einen neunzehnten Schritt, bei dem das Produkt aus dem achtzehnten Schritt zentrifugiert und unter Verwendung einer Korbzentrifuge filtriert wird und Cholesterinkristalle mit einer Reinheit von mehr als 95% erhalten werden.

4. Verfahren zur Cholesterinabtrennung in der schweren Fraktion von Fischöl gemäß den Ansprüchen 1, 2 und 3, wobei das Waschlösungsmittel aus einer Lösung von Natriumhydroxid oder Calciumchlorid ausgewählt werden kann.

## Revendications

1. Procédé de séparation de cholestérol de la fraction lourde d'huile de poisson et **caractérisé en ce qu'il** comprend une première étape d'obtention d'acides gras sous la forme de leurs esters et comprenant :
(i) une première étape de transestérification d'huile de poisson raffinée ayant un indice d'acide inférieur à 2 mg KOH/g à partir de la réaction avec du méthanol, du propanol, du butanol ou de l'éthanol dans un rapport compris entre 1:5 et 1:2 p/p par rapport à l'huile raffinée, de préférence 1:3 p/p, avec l'ajout d'un catalyseur basique choisi dans le groupe constitué d'hydroxyde de sodium, hydroxyde de potassium, éthanolate de sodium ou méthanolate de sodium à une concentration comprise entre 0,75 et 1,5 % p/p par rapport à l'huile, où il est laissé réagir dans quatre réacteurs à ultrasons agencés en série entre 20 et 60 minutes, à une pression comprise entre 1 et 3 bar, à une température comprise entre 60 °C et 90 °C et à reflux constant d'alcool ;
(ii) une deuxième étape pour récupérer l'alcool non consommé dans la réaction par évaporation instantanée dans un évaporateur qui fonctionne à une pression comprise entre 0,2 et 1 bar et à une température comprise entre 50 et 80 °C, où l'alcool récupéré dans cette étape peut être réutilisé dans la première étape ;
(iii) une troisième étape pour séparer le mélange de l'ester et du glycérol obtenu par la transestérification au moyen d'une centrifugeuse à disque qui fonctionne entre 3 500 et 6 000 tours/minute, dans lequel la réaction atteint une conversion des glycérides en esters d'au moins 95 % ;
(iv) une quatrième étape de concentration du cholestérol présent dans l'huile sous la forme d'ester par distillation moléculaire, obtenant une fraction lourde, à l'aide de distillateurs à court trajet agencés en série, où le résidu provenant d'un évaporateur est introduit dans le distillateur suivant et le distillat est renvoyé dans le distillateur précédent, où les conditions de fonctionnement des distillateurs comprennent des pressions de vide allant de 0,1 à 10 Pa, des températures comprises entre 120 et 165 °C et des surfaces de transfert de chaleur comprises entre 2 et 5 m² et dans lequel, au cours de cette étape, un courant concentré en teneur en cholestérol sous la forme d'ester et un autre courant d'esters concentrés en acides gras EPA et DHA sont obtenus.

2. Procédé de séparation de cholestérol de la fraction lourde d'huile de poisson selon la revendication 1, comprenant une deuxième étape d'obtention de produits de saponification et d'extraction qui comporte :
(v) une cinquième étape pour obtenir un savon à l'aide d'une base choisie dans le groupe constitué d'hydroxyde de calcium, hydroxyde de sodium, hydroxyde de potassium ou chlorure de calcium dans l'eau à une concentration comprise entre 10 % et 30 % p/p, avec l'ajout d'éthanol, de méthanol, de glycérol ou de propylène glycol dans un rapport compris entre 2 et 5 % p/p, de préférence 3 % p/p, par rapport à la solution de base dans un mélangeur à haut rendement, où il est mélangé avec la fraction concentrée de cholestérol dans un rapport en poids compris entre 1:0,9 ; ou 1:1,1 ; ou 1:1,2, de préférence 1:1, par rapport au courant provenant de la distillation moléculaire et où le mélange entre dans un réacteur où il est saponifié pendant une durée comprise entre 1 et 4 heures, de préférence 1 à 2 heures, à une température comprise entre 60 °C et 85 °C avec une agitation comprise entre 500 et 1 050 tours/minute à l'aide d'un reflux constant ;
(vi) une sixième étape d'extraction où le savon provenant de la cinquième étape entre dans une colonne d'extraction, dans laquelle un solvant choisi dans le groupe constitué de toluène, dichlorure d'éthylène, chlorure de méthylène, de préférence le dichlorure d'éthylène, est ajouté, dans un rapport de 4:1 à 1:1, de préférence 3:1, par rapport au savon introduit, avec une agitation constante comprise entre 200 tours/minute et 750 tours/minute, et à une température comprise entre 30 et 50 °C ;
(vii) une septième étape consistant à favoriser une séparation ultérieure de phases qui comporte l'ajout d'éthanol, de méthanol, de propanol ou de butanol à une concentration de 0 à 5 % en poids par rapport au mélange du solvant d'extraction et du savon ;
(viii) une huitième étape qui comporte la centrifugation du mélange d'extraction pour la séparation du savon et du solvant à une plage de vitesses comprise entre 500 et 3 500 tours/minute ;
(ix) une neuvième étape qui comprend une nouvelle extraction du savon centrifugé provenant de la huitième étape dans une colonne d'extraction, dans laquelle sont ajoutés de l'éther diéthylique, de l'éther de pétrole, de l'acétone, du benzène ou de l'hexane, dans un rapport de 4:1 à 1:1, par rapport au savon introduit, avec une agitation constante et à une température comprise entre 30 et 55 °C et accompagnée du fait de favoriser la séparation des phases de la septième étape, comportant également la centrifugation du mélange d'extraction sur la base de la huitième étape ;
(x) une dixième étape qui comporte la réalisation des étapes six à neuf 1 à 3 fois supplémentaires ;
(xi) une onzième étape où le solvant provenant des phases de dispersion de chacune des centrifugations envisagées aux huitième, neuvième et dixième étapes est évaporé et récupéré au moyen d'une batterie d'évaporateurs agencés en série, qui fonctionnent à des pressions allant de 10 à 100 Pa et à des températures comprises entre 30 et 120 °C, dans lequel, après évaporation, la matière insaponifiable (MI) qui a été dissoute dans le solvant est obtenue ;
(xii) une douzième étape où le savon obtenu par la dernière centrifugation de la dixième étape est disposé pour être acidifié avec un acide choisi dans le groupe constitué d'acide sulfurique, acide chlorhydrique, acide acétique, acide phosphorique ou acide citrique dans une solution aqueuse à 50 à 70 % p/p dans un réacteur à ultrasons, où le rapport entre le savon et la solution acide est compris entre 3:1 et 1:1, de préférence 2:1, et à une température comprise entre 70 °C et 90 °C pendant 1 à 3 heures sous agitation constante, puis il est refroidi et l'eau est séparée des acides gras libres formés à l'aide d'une centrifugeuse qui fonctionne dans une plage comprise entre 450 et 850 tours/minute ;
(xiii) une treizième étape qui envisage l'estérification des acides gras libres avec des ultrasons à l'aide d'éthanol, de méthanol ou de propanol par rapport à l'acide gras libre en poids entre 1:5 à 1:3, de préférence 1:4, et un catalyseur acide tel que l'acide sulfurique, l'acide p-toluènesulfonique, l'acide citrique, l'acide acétique, l'acide phosphorique ou l'acide chlorhydrique dissous dans de l'alcool à une concentration de 3 à 8 % p/p pendant une à trois heures à une température comprise entre 65 °C et 85 °C sous agitation et à reflux constant et, enfin, l'obtention d'un courant d'acides gras sous la forme de leurs esters.

3. Procédé de séparation de cholestérol de la fraction lourde d'huile de poisson selon les revendications 1 et 2, comprenant une troisième étape de purification et de cristallisation de cholestérol qui comporte :
(xiv) une quatorzième étape où la matière insaponifiable est lavée avec une solution aqueuse d'hydroxyde de sodium ou de chlorure de calcium à 5 à 10 % ; dans lequel le lavage est effectué dans une proportion de 1:2 et 1:4 en poids par rapport à la matière insaponifiable désolvantisée, à une plage de températures comprise entre 40 et 60 °C ;
(xv) une quinzième étape qui comporte la centrifugation du mélange obtenu après lavage pour séparer la matière insaponifiable traitée et l'eau de lavage, laquelle étape est exécutée à une plage de vitesses comprise entre 500 et 3 500 tours/minute, de préférence à 2 000 tours/minute ;
(xvi) une seizième étape où un solvant organique choisi parmi acétone, benzène ou toluène est ajouté à la matière insaponifiable provenant de la quinzième étape dans un rapport de 15:1 à 5:1, chauffé à 30 °C et agité jusqu'à dissolution, puis cristallisé à une température comprise entre -10 °C et 5 °C. et maintenu dans des conditions isothermes à 5 °C pendant 8 heures, puis refroidi à -10 °C pendant 10 heures ;
(xvii) une dix-septième étape où, par la suite, le produit résultant provenant de la seizième étape est centrifugé et filtré à l'aide d'une centrifugeuse à panier pour obtenir des cristaux de cholestérol ayant une pureté supérieure à 70 % ;
(xviii) une dix-huitième étape où un autre solvant organique choisi parmi un éther diéthylique, l'éther de pétrole ou l'hexane est ajouté au cholestérol à 70 % provenant de la dix-septième étape dans un rapport de 15:1 à 5:1, chauffé à 30 °C et agité jusqu'à dissolution, puis cristallisé à une température comprise entre - 40 °C et 0 °C, puis maintenu dans des conditions isothermes à -5 °C pendant 10 heures, puis refroidi à -40 °C pendant 8 heures ; et
(xix) une dix-neuvième étape où le produit provenant de la dix-huitième étape est centrifugé et filtré à l'aide d'une centrifugeuse à panier et des cristaux de cholestérol ayant une pureté supérieure à 95 % sont obtenus.

4. Procédé de séparation de cholestérol présent dans la fraction lourde d'huile de poisson selon les revendications 1, 2 et 3, dans lequel le solvant de lavage peut être choisi parmi une solution d'hydroxyde de sodium ou de chlorure de calcium.
